# EUROPEAN PATENT SPECIFICATION

(11) **EP 2 232 299 B1**
(45) Date of publication and mention of the grant of the patent: **15.02.2012**
(21) Application number: 08857737.4
(22) Date of filing: 08.12.2008
(51) Int. Cl.: G01S 15/89, G01S 7/52, A61B 8/06, G10K 11/34

(54) **METHOD AND SYSTEM FOR IMAGING VESSELS**
VERFAHREN UND SYSTEM ZUR BILDGEBUNG VON GEFÄSSEN
PROCEDE ET SYSTEME D'IMAGERIE DE VAISSEAUX

(30) Priority: 07.12.2007 US 12071 P
(43) Date of publication of application: 29.09.2010
(73) Proprietor: Koninklijke Philips Electronics N.V., 5621 BA Eindhoven (NL)
(72) Inventor: SHI, Xuegong, Briarcliff Manor New York 10510-8001 (US)
(74) Representative: Van Velzen, Maaike Mathilde
(86) International application number: PCT/IB2008/055150
(87) International publication number: WO 2009/072092

(56) References cited:
- US-A- 6 048 314
- US-A1- 2004 267 127
- US-A1- 2006 122 513

## Description

### FIELD OF THE INVENTION

This disclosure relates generally to imaging systems and more specifically to a method and system for imaging vessels.

### BACKGROUND

Imaging of vessels can be based upon detecting blood flow through the vessels. For example, Transcranial Doppler is a test that measures the velocity of blood flow through the brain's blood vessels, such as to diagnosis emboli, stenosis, vasospasm from a subarachnoid hemorrhage, and other problems.

Transcranial Doppler can be performed utilizing "B-mode" imaging, which displays a 2-dimensional image as seen by the ultrasound probe. Once the operator is able to find the desired blood vessel, blood flow velocities may be measured with a pulsed Doppler probe, which graphs velocities over time. Together, these make a duplex test. A second method of recording can use only the second probe function, and again can rely on the training and experience of the operator in finding the correct vessels. A cerebrovascular exam can follow a standard protocol starting with examination of the middle cerebral artery, progressing through the anterior and posterior cerebral arteries, and finishing with the terminal internal carotid artery.

Blood flow velocity can be recorded by emitting a high-pitched sound wave from the ultrasound probe, which then bounces off of various materials to be measured by the same probe. A specific frequency can be used, and the speed of the blood in relation to the probe causes a phase shift, with the frequency being increased or decreased. This frequency change correlates with the speed of the blood, which is then recorded electronically for later analysis. A range of depths and angles must be measured to ascertain the correct velocities, as recording from an angle to the blood vessel yields an artificially low velocity.

The bones of the skull can block transmissions of ultrasound waves. Thus, an operator must utilize small and specifically located acoustic windows on human skulls. Additionally, the cerebral vessels, such as along the Circle of Willis, have a tortuous path that require the operator to be continuously tilting and rotating the ultrasound transducer while moving the Doppler sampling volume location in order to examine the cerebral vessels. The training and practice to master these operator techniques may be hindering the use of Transcranial Doppler exams. Additionally, the awkward and strenuous positions of the sonographers hand and arms during the examination can result in musculoskeletal injuries.

Accordingly, there is a need for a method and system for imaging vessels that facilitates capturing images and mapping vessel flow.

US 2004/0267127, which is considered as closest prior art document, discloses an ultrasound imaging method a system that permit three-dimensional imaging of blood flow. A processor collects and Doppler processes ultrasound blood velocity data in a three dimensional region through the use of a planar phased array of piezoelectric elements. The processor locks onto and tracks the points in three-dimensional space that produce the locally maximum blood velocity signals. The integrated coordinates of points acquired by the accurate tracking process is used to form a three-dimensional map of blood vessels.

### SUMMARY

In one exemplary embodiment of the present disclosure, a method of imaging of vessels according to claim 1 is provided.

In another exemplary embodiment, a method of performing transcranial imaging according to claim 8.

In a further exemplary embodiment, an ultrasound imaging system according to claim 12 is provided.

The technical effect includes, but is not limited to, facilitating the capture of data and images for mapping vessel flow. The technical effect further includes, but is not limited to, reducing or eliminating stress and injuries for the sonographic operators performing an ultrasonic vessel exam.

The above-described and other features and advantages of the present disclosure will be appreciated and understood by those skilled in the art from the following detailed description, drawings, and appended claims.

### BRIEF DESCRIPTION OF THE DRAWINGS

Figure 1 is a schematic illustration of a system for performing imaging of vessels according to an exemplary embodiment of the present invention;

Figure 2 is a schematic illustration of a series of vessels that can be imaged by system of FIG. 1; and

Figure 3 is a method that can be used by the system of FIG. 1 for performing vessel imaging according to an exemplary embodiment of the present invention.

### DETAILED DESCRIPTION OF THE INVENTION

The exemplary embodiments of the present disclosure are described with respect to data capture, vessel imaging and blood flow mapping for a Transcranial Doppler exam of the Circle of Willis of a human. It should be understood by one of ordinary skill in the art that the exemplary embodiments of the present disclosure can be applied to vessels of other portions of the body, whether human or animal. The use of the method and system of the exemplary embodiments of the present disclosure can be adapted for application to vessels other than of the Circle of Willis utilizing a number of techniques, including adjusting the physiological parameters employed in the Transcranial Doppler example to correspond to the physiological parameters associated with the other vessels, including depth and path.

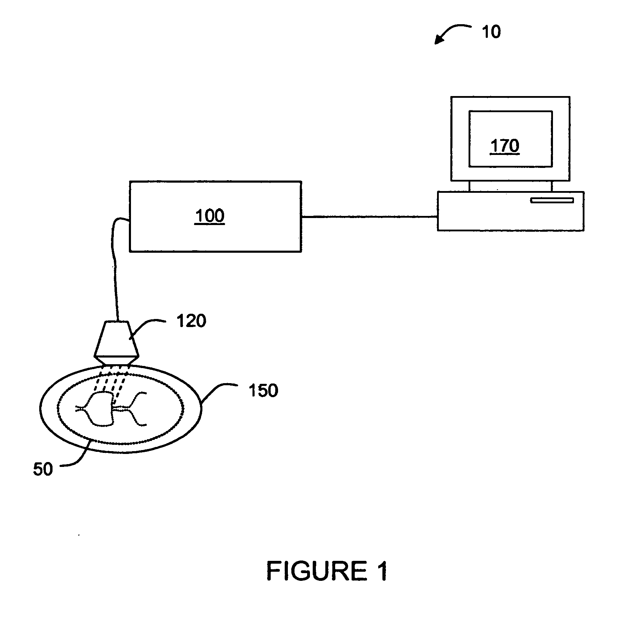

Referring to the drawings, and in particular to FIG. 1, an ultrasound imaging system in accordance with one exemplary embodiment of the invention is shown and generally represented by reference numeral 10. System 10 can perform ultrasound imaging on a patient's head 50, and can include a processor or other control device 100, a probe or transducer 120, a support structure 150, and a display device 170.

Processor 100 can include various components for performing ultrasound imaging, and can employ various imaging techniques, such as with respect to data capture, analysis and presentation. For example, the processor 100 can include a beamformer for processing received echo signals, a Doppler processor for processing Doppler-related information, and an image processor for forming 2D or 3D images. The processor 100 can also include a memory device, such as a CINELOOP ® memory, and a video processor. The processor 100 can include components and/or techniques associated with the steering and electronic focusing of the ultrasound waves of the probe 120, as described more particularly below. Other components and/or techniques can also be used with the processor 100, such as an automatic border detection processor that can define and graphically overlay anatomical borders with respect to the images presented. The present disclosure also contemplates the use of other components and/or techniques in addition to, or in place of, the components of processor 100 described above.

Probe 120 can include an array of transducer or acoustic elements for the transmission of ultrasonic waves and for the receipt of ultrasonic echo signals. Probe 120 allows for steering and electronic focusing of the ultrasound waves with respect to the vessels under examination. For example, probe 120 can include a transmit/receive (T/R) switch coupled to the transducer array, such as a two-dimensional array of transducer elements for performing three-dimensional scanning. The transducer array can transmit ultrasound energy into a region being imaged and receive reflected ultrasound energy or echos, from the vessels and, in particular, the blood flow, as well as from various other structures and organs within the patient's body. By appropriately delaying the pulses applied to each transducer element, the probe 120 can transmit a focused ultrasound beam along a desired transmit scan line.

According to one embodiment, the array transducer of the probe 120 can include a two dimensional array such as disclosed in U.S. Pat No. 6,428,477, assigned to the assignee of the present disclosure . U.S. Pat. No. 6,428,477 discloses delivery of therapeutic ultrasound and performing ultrasound diagnostic imaging with the use of a two dimensional ultrasound array. The two dimensional ultrasound array includes a matrix or grid of transducer elements that allows three-dimensional (3D) images to be acquired, although 2D imaging is also contemplated by the present disclosure. The matrix of transducer elements makes possible the steering and electronic focusing of ultrasound energy in any arbitrary direction.

The transducer array of probe 120 can be coupled to an ultrasound receiver through the T/R switch. Reflected ultrasound energy from a given point within the patient's body can be received by the transducer elements at different times. The transducer elements of the probe 120 can convert the received ultrasound energy to received electrical signals which are amplified by the receiver and are supplied to a receive beamformer. The signals from each transducer element can be individually delayed and then can be summed by the beamformer to provide a beamformer signal that is a representation of the reflected ultrasound energy level along a given receive scan line. The delays applied to the received signals may be varied during reception of ultrasound energy to effect dynamic focusing. The process can be repeated for multiple scan lines to provide signals for generating an image of a region of interest in the patient's body. Because the transducer array is two-dimensional, the receive scan lines can be steered in azimuth and in elevation to form a three-dimensional scan pattern. The beamformer may, for example, be a digital beamformer such as may be found in any suitable commercially available medical diagnostic ultrasound machine.

The beamformer signals can be stored in an image data buffer of the system 10, which stores image data for different volume segments of an image volume and for different points of a cardiac cycle. The image data can be output from image data buffer to the display device 170, which generates a three-dimensional image of the region of interest from the image data. The display device 170 may include a scan converter which converts sector scan signals from the beamformer to conventional raster scan display signals. Controller 100 can provide overall control of the ultrasound diagnostic imaging system, including timing and control functions.

In one embodiment, probe 120 can be connected to the support structure or helmet 150 via a connection structure. The type of connection structure can vary. For example, the probe 120 can be removably connectable with the helmet 150, such as on one or both sides of the helmet in proximity to the temporal region of the skull. In another embodiment, the connection structure can be adjustable so that the positioning of the probe 120 can be adjusted with respect to the patient's head 50. A hole or opening can be provided in the helmet 150 for positioning of the probe 120 therein or the probe can be connected to an outer surface of the helmet, which may be made of material that allows for passage of the ultrasound waves therethrough. In one embodiment, the helmet 150 allows for connection of various types of probes 120 thereto in order to enable the system 10 to be retrofitted to existing ultrasound hardware components.

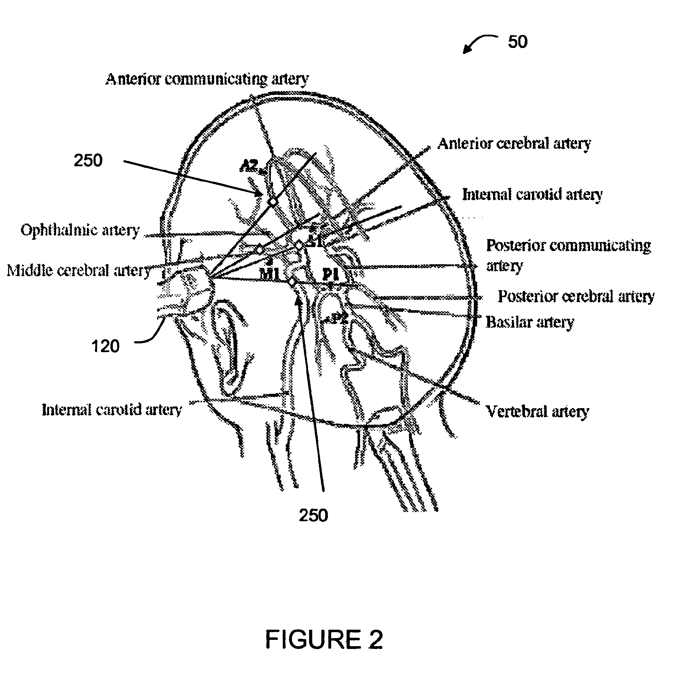

The present disclosure contemplates the probe 120 being used without the helmet 150 or with a modified support structure. For example, as shown in FIG. 2, the probe 120 can be held by the operator in place through the exam in proximity to the temporal region of the patient's head. In another embodiment, the support structure 150 can be a strap or support member that can be placed over a portion of the patient's head 50 to position the probe 120 as desired without enclosing the patient's head. Other structures and techniques are also contemplated for positioning of the probe 120 with respect to the patient's head 50, including a bed with a fixed probe against which the patient can place his or her head.

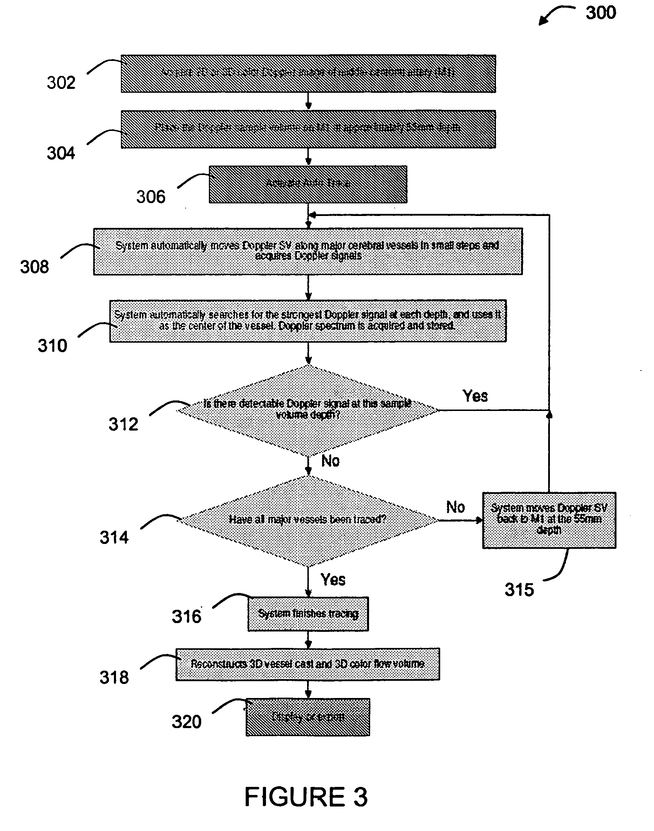

Referring additionally to FIG. 3, an exemplary method of operation of the system 10 is shown and generally represented by reference numeral 300. It would be apparent to an artisan with ordinary skill in the art that other embodiments not depicted in FIG. 3 are possible without departing from the scope of the claims described below, including examination of other portions of the body. Method 300 can provide for a 3D vessel cast and 3D flow volume map in and around the Circle of Willis, which is shown in FIG. 2 and includes vessels such as the middle cerebral artery, ophthalmic artery, anterior communicating artery, anterior cerebral artery, internal carotid artery, posterior communicating artery, posterior cerebral artery, basilar artery, and vertebral artery.

Method 300 begins with step 302 in which a Doppler image, such as a color 2D or 3D image, is obtained of the middle cerebral artery by positioning of the probe 120 with respect to the helmet 150 and in proximity to the temporal window of the patient's head 50. The present disclosure also contemplates the initial vessel to be examined to be other than the middle cerebral artery.

A Doppler sample volume for the system 10 can then be set to a 55 mm depth in step 304. The depth of 55 mm is based upon a typical location of the middle cerebral artery in a human head. The actual depth used by the operator can also be varied based on a number of factors, such as age, skull measurements, and so forth. In step 306, an operator can actuate the tracing algorithm for the first vessel. The actuation can be by a number of techniques including pushing a button or voice activation.

Method 300 can trace the middle cerebral artery by moving the Doppler sample volume shallower, i.e., decreasing the depth, along the middle cerebral artery, such as in 1 mm increments. The color Doppler image can be used to guide the steering of the Doppler beam and the placement of the Doppler sample volume. At each new sample volume position, the strongest Doppler signal will be determined and can be utilized as the center point or center line of the vessel at that depth, as in step 310. The probe 120 can then steer the ultrasound energy at that sample volume depth to capture blood flow data and determine the boundaries of the vessel (i.e., the vessel walls) at that sample volume depth. For example, the search region can be a 2 x 2 or 3 x 3 mm grid in the C-place, although other search regions are contemplated. The measurement of the Doppler signal can be performed over one or more cardiac cycles, such as based on the integrated Doppler power or peak flow velocity. The use of at least a full cardiac cycle allows for a full representation of the flow dynamics.

In step 312, the system 10 moves to the next sample volume depth and determines if there is a detectable Doppler signal. If a detectable signal exists then system 10 repeats steps 308 and 310 to capture data of the blood flow and boundaries of the vessel at that sample volume depth. If there is no detectable Doppler signal, then system 10 can determine if all vessels that are to be examined have been traced as in step 314.

In step 315, where no detectable Doppler signal exists for the particular sample volume depth then system 10 returns the sample volume to the 55 mm depth along the middle cerebral artery. The sample volume depth can then be increased, such as in 1 mm increments and the data capturing steps 308 and 310 can be repeated for the series of increasing depths along the middle cerebral artery.

To continue to trace along the Circle of Willis, method 300 can repeat the above steps of capturing data, changing sample volume depth and searching for a detectable Doppler signal at the new sample volume depth. At various points along the Circle of Willis, the Doppler signal will no longer be detectable and the system 100 can return the previous sample volume depth starting point for that particular vessel. For example, after moving along the middle cerebral artery in increasing depths, the trace will reach the bifurcation point of the middle and anterior cerebral arteries where the Doppler spectrum becomes bi-directional. System 10 can continue tracing the anterior cerebral artery by moving the sample volume depth deeper and anteriorly along the bifurcation until no Doppler signal can be detected. System 10 can then return the sample volume to the bifurcation point and start tracing deeper and posteriorly along the posterior cerebral artery until no Doppler signal can be detected.

In one embodiment, typical physiological measurements can be used to assist in determining vessel positioning and steering of the ultrasound waves. For example, method 300 can confirm that a Doppler signal is no longer detectable and that the previous sample volume starting point should be returned to based upon a typical location of the vessel in the human head, such as the bifurcation point of the middle and anterior cerebral arteries being between 60 and 70 mm deep.

Once all of the desired vessels have been traced, system 10 can fmish tracing in step 316 and begin reconstructing in step 318 a 3D vessel cast and a 3D flow volume map. In step 320, the images and/or data can be displayed, such as on display device 320 or can be exported elsewhere.

The 3D reconstruction of the vessel map can include connecting the sample volume positions to build a vessel skeleton; integrating the Doppler power data of each sample volume position; adjusting the brightness of the vessel skeleton to represent the Doppler power of each sample volume point; and interpolating and smoothing between the sample volume points to generate an angiograph-like vessel graph. The generation of the 3D flow map can include calculating the Doppler mean velocity of each sample volume position; synchronizing the mean velocity of all sample volume points using the arterial pulsatility in the Doppler spectrum; and applying Doppler angle correction using the vessel orientation. The mean velocity can be calculated at every point where the Doppler spectrum has been acquired. The synchronization may not be required when the pulsatility cannot be detected in the Doppler spectrum.

As shown in FIG. 2, the probe 120 can provide electronically steered Doppler beams with sample volumes 250 being taken along various depths with respect to the vessels. Doppler signals can then be captured and analyzed at each sample volume position. The color 3D Doppler image can be used to guide the placement of the sample volume. The Doppler spectrum can be acquired and stored along the trace to provide real-time display and reconstruct a 3D vessel cast. In one embodiment, the system 10 can retrieve and display the Doppler spectrum in post-acquisition review when the operator or other user places a cursor on the vessel in the 3D vessel cast.

For a Transcranial Doppler examination, the present disclosure describes the 3D vessel cast and flow volume map being generated using a single probe 120 positioned along only one side of the patient's head 50. The present disclosure also contemplates the use of two probes positioned on opposing sides of the patient's head or a single probe that gathers data from one side of the patient's head and then is moved to the other side for capturing of the data.

The invention, including the steps of the methodologies described above, can be realized in hardware, software, or a combination of hardware and software. The invention can be realized in a centralized fashion in one computer system, or in a distributed fashion where different elements are spread across several interconnected computer systems. Any kind of computer system or other apparatus adapted for carrying out the methods described herein is suited. A typical combination of hardware and software can be a general purpose computer system with a computer program that, when being loaded and executed, controls the computer system such that it carries out the methods described herein.

The invention, including the steps of the methodologies described above, can be embedded in a computer program product. The computer program product can comprise a computer-readable storage medium in which is embedded a computer program comprising computer-executable code for directing a computing device or computer-based system to perform the various procedures, processes and methods described herein. Computer program in the present context means any expression, in any language, code or notation, of a set of instructions intended to cause a system having an information processing capability to perform a particular function either directly or after either or both of the following: a) conversion to another language, code or notation; b) reproduction in a different material form.

The illustrations of embodiments described herein are intended to provide a general understanding of the structure of various embodiments, and they are not intended to serve as a complete description of all the elements and features of apparatus and systems that might make use of the structures described herein. Many other embodiments will be apparent to those of skill in the art upon reviewing the above description. Other embodiments may be utilized and derived therefrom, such that structural and logical substitutions and changes may be made without departing from the scope of this disclosure. Figures are also merely representational and may not be drawn to scale. Certain proportions thereof may be exaggerated, while others may be minimized. Accordingly, the specification and drawings are to be regarded in an illustrative rather than a restrictive sense.

Thus, although specific embodiments have been illustrated and described herein, it should be appreciated that any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description. Therefore, it is intended that the disclosure not be limited to the particular embodiment(s) disclosed as the best mode contemplated for carrying out this invention, but that the invention will include all embodiments falling within the scope of the appended claims.

## Claims

1. A method of imaging vessels, the Method comprising:
positioning an ultrasound probe (120) proximate a temporal window of a body to acquire a Doppler image of an initial vessel to be examined;
transmitting ultrasonic waves into a corresponding region of the body having the vessel and receiving echoes in response, the echoes being associated with blood flow through the vessel;
adjusting positions of sample volumes (250) associated with the echoes subsequent to receiving echoes in an initial sample volume, the initial sample volume being set at an initial position and depth based upon a typical location of the initial vessel in the body, the adjusting of the subsequent positions of the sample volumes being based on a tracing algorithm for tracing a path of the vessel by moving the positions of subsequent sample volumes in increments of depth along the vessel;
electronically steering the ultrasonic waves at the subsequent positions of the sample volumes; and
determining a wall of the vessel at each of the subsequent positions of the sample volumes based on a Doppler spectrum captured at each of the subsequent positions of the sample volumes.

2. The method of claim 1, further comprising:
adjusting the subsequent positions of sample volumes in increments along a first direction until a strength of a Doppler signal is below a threshold; and
returning to a first position of the subsequent positions of the sample volumes when the strength of the Doppler signal has fallen below the threshold.

3. The method of claim 2, further comprising adjusting the subsequent positions of the sample volumes in increments along a second direction until a strength of the Doppler signal is below the threshold, the second direction being opposite to the first direction.

4. The method of claim 1, further comprising:
constructing a vessel map by connecting at least a portion of the initial and subsequent positions of the sample volumes to build a vessel skeleton;
integrating Doppler power data of each of the at least a portion of the initial and subsequent positions of the sample volumes along the vessel skeleton; and
adjusting a brightness of the vessel skeleton to represent the Doppler power data.

5. The method of claim 4, further comprising at least one of interpolating and smoothing between points along the vessel skeleton.

6. The method of claim 4, further comprising:
calculating a Doppler mean velocity of each of the at least a portion of the initial and subsequent positions of the sample volumes; and
synchronizing the mean velocity using an arterial pulsatility in the Doppler spectrum when the pulsatility is detected.

7. The method of claim 6, further comprising applying Doppler angle correction using an orientation of the vessel.

8. A method of performing transcranial imaging, the method comprising:
acquiring a Doppler image of an initial vessel in the transcranial region;
positioning an initial Doppler sample volume (250) in proximity to the initial vessel at a pre-determined depth using the Doppler image as a guide;
adjusting positions of subsequent sample volumes based on a tracing algorithm for tracing a path of the vessel by moving the positions of the subsequent sample volumes in increments of depth along the vessel;
electronically steering ultrasonic waves at the positions of the subsequent sample volumes; and
determining a center line and a wall of the vessel for at least a portion of the positions of the subsequent sample volumes based on a Doppler spectrum associated with blood flow through the vessel that is captured at each of the positions of the subsequent sample volumes, the determination of the center line and the wall being determined based on a strength of the Doppler signal.

9. The method of claim 8, further comprising:
constructing a vessel map by connecting at least a portion of the positions of the subsequent sample volumes to build a vessel skeleton;
integrating Doppler power data of each of the at least a portion of the positions of the subsequent sample volumes along the vessel skeleton; and
adjusting a brightness of the vessel skeleton to represent the Doppler power data.

10. The method of claim 8, further comprising:
adjusting the positions of the subsequent sample volumes in increments along a first direction until a Doppler signal is no longer detected;
returning to a first position of the sample volumes associated with the predetermined depth when the Doppler signal is no longer detected; and
adjusting the positions of the subsequent sample volumes in increments along a second direction until a Doppler signal is no longer detected, the second direction being opposite to the first direction.

11. The method of claim 8, further comprising:
calculating a Doppler mean velocity of each of the at least a portion of the positions of the subsequent sample volumes;
synchronizing the mean velocity using an arterial pulsatility in the Doppler spectrum when the pulsatility is detected; and
applying Doppler angle correction using an orientation of the vessel.

12. An ultrasound imaging system (10) comprising:
a matrix transducer array (120) for transmitting ultrasonic waves into a region of a body having a vessel and receiving echoes in response, the echoes being associated with blood flow through the vessel; and
a processor (100) operably coupled to the matrix transducer array, wherein the processor adjusts positions of sample volumes (250) associated with the echoes subsequent to receiving echoes in an initial sample volume, the initial sample volume being set at an initial position and depth based upon a typical location of the initial vessel in the body, the adjusting of the subsequent positions of the sample volumes being based on a tracing algorithm for tracing a path of the vessel by moving the positions of subsequent sample volumes in increments of depth along the vessel, wherein the processor electronically steers the ultrasonic waves at the subsequent positions of the sample volumes, and wherein the processor determines a wall of the vessel at each of the subsequent positions of the sample volumes based on a Doppler spectrum captured at each of the subsequent positions of the sample volumes.

13. The system of claim 12, wherein the processor determines a center line of the vessel based on a strength of a Doppler signal at each of the subsequent positions of the sample volumes.

14. The system of claim 12, further comprising a support structure (150) for positioning the matrix transducer array (120) with respect to the region of the body having the vessel.

15. The system of claim 12, further comprising a display device (170) in communication with the processor (100), wherein the display device presents a vessel map generated by the processor based at least in part on the determination of the wall of the vessel at each of the initial and subsequent positions of the sample volumes (250).

## Patentansprüche

1. Verfahren zur Bildgebung von Gefäßen, wobei das Verfahren Folgendes umfasst:
Positionieren einer Ultraschallsonde (120) proximal zu einem zeitlichen Fenster eines Körpers, um ein Doppler-Bild eines zu untersuchenden anfänglichen Gefäßes zu erfassen;
Aussenden von Ultraschallwellen in eine entsprechende Region des das Gefäß enthaltenden Körpers und Empfangen von Echos in Reaktion darauf, wobei die Echos der Blutströmung durch das Gefäß zugehörig sind;
Anpassen von zu den Echos gehörigen Positionen der Abtastvolumina (250) im Anschluss an das Empfangen von Echos in einem anfänglichen Abtastvolumen, wobei das anfängliche Abtastvolumen basierend auf einer typischen Lage des anfänglichen Gefäßes im Körper auf eine Anfangsposition und -tiefe eingestellt ist, wobei das Anpassen der nachfolgenden Positionen der Abtastvolumina auf einem Verfolgungsalgorithmus zum Verfolgen eines Pfads des Gefäßes durch Bewegen der Positionen der nachfolgenden Abtastvolumina in Tiefe-Inkrementen entlang des Gefäßes basiert;
elektronisches Lenken der Ultraschallwellen auf die nachfolgenden Positionen der Abtastvolumina; und
Ermitteln einer Gefäßwand bei jeder der nachfolgenden Positionen der Abtastvolumina basierend auf einem Doppler-Spektrum, das bei jeder der nachfolgenden Positionen der Abtastvolumina erfasst wurde.

2. Verfahren nach Anspruch 1, das weiterhin Folgendes umfasst:
Anpassen der nachfolgenden Positionen von Abtastvolumina in Inkrementen entlang einer ersten Richtung, bis eine Stärke eines Doppler-Signals unter einem Schwellenwert liegt; und
Zurückkehren zu einer ersten Position der nachfolgenden Positionen der Abtastvolumina, wenn die Stärke des Doppler-Signals unter den Schwellenwert abgefallen ist.

3. Verfahren nach Anspruch 2, das weiterhin das Anpassen der nachfolgenden Positionen der Abtastvolumina in Inkrementen entlang einer zweiten Richtung umfasst, bis eine Stärke des Doppler-Signals unter dem Schwellenwert liegt, wobei die zweite Richtung der ersten Richtung entgegengesetzt ist.

4. Verfahren nach Anspruch 1, das weiterhin Folgendes umfasst:
Erstellen einer Gefäßkarte durch Verbinden von mindestens einem Teil der anfänglichen und nachfolgenden Positionen der Abtastvolumina, um ein Gefäßskelett zu bilden;
Integrieren der Doppler-Leistungsdaten von jedem der mindestens einen Teile der anfänglichen und nachfolgenden Positionen der Abtastvolumina entlang des Gefäßskeletts; und
Anpassen einer Helligkeit des Gefäßskeletts zur Darstellung der Doppler-Leistungsdaten.

5. Verfahren nach Anspruch 4, das weiterhin mindestens entweder das Interpolieren oder das Glätten zwischen Punkten entlang des Gefäßskeletts umfasst.

6. Verfahren nach Anspruch 4, das weiterhin Folgendes umfasst:
Berechnen einer mittleren Doppler-Geschwindigkeit von jedem der mindestens einen Teile der anfänglichen und nachfolgenden Positionen der Abtastvolumina; und
Synchronisieren der mittleren Geschwindigkeit unter Verwendung einer arteriellen Pulsatilität in dem Doppler-Spektrum, wenn die Pulsatilität detektiert wird.

7. Verfahren nach Anspruch 6, das weiterhin das Anwenden von Doppler-Winkelkorrektur unter Verwendung einer Orientierung des Gefäßes umfasst.

8. Verfahren zur Durchführung einer transkranialen Bildgebung, wobei das Verfahren Folgendes umfasst:
Erfassen eines Doppler-Bildes eines anfänglichen Gefäßes in der transkranialen Region;
Positionieren eines anfänglichen Doppler-Abtastvolumens (250) in der Nähe des anfänglichen Gefäßes in einer vorgegebenen Tiefe unter Verwendung des Doppler-Bildes als Führung;
Anpassen der Positionen von nachfolgenden Abtastvolumina basierend auf einem Verfolgungsalgorithmus zum Verfolgen eines Pfads des Gefäßes durch Bewegen der Positionen der nachfolgenden Abtastvolumina in Tiefe-Inkrementen entlang des Gefäßes;
elektronisches Lenken der Ultraschallwellen auf die Positionen der nachfolgenden Abtastvolumina; und
Ermitteln einer Mittellinie und einer Wand des Gefäßes für mindestens einen Teil der Positionen der nachfolgenden Abtastvolumina basierend auf einem zu der Blutströmung durch das Gefäß gehörigem Doppler-Spektrum, das bei jeder der Positionen der nachfolgenden Abtastvolumina erfasst wurde, wobei die Ermittlung der Mittellinie und der ermittelten Wand auf einer Stärke des Doppler-Signals basiert.

9. Verfahren nach Anspruch 8, das weiterhin Folgendes umfasst:
Erstellen einer Gefäßkarte durch Verbinden mindestens eines Teils der Positionen der nachfolgenden Abtastvolumina, um ein Gefäßskelett zu bilden;
Integrieren der Doppler-Leistungsdaten von jedem der mindestens einen Teile der Positionen der nachfolgenden Abtastvolumina entlang des Gefäßskeletts; und
Anpassen einer Helligkeit des Gefäßskeletts zur Darstellung der Doppler-Leistungsdaten.

10. Verfahren nach Anspruch 8, das weiterhin Folgendes umfasst:
Anpassen der Positionen der nachfolgenden Abtastvolumina in Inkrementen entlang einer ersten Richtung, bis ein Doppler-Signal nicht mehr detektiert wird;
Zurückkehren zu einer ersten Position der Abtastvolumina, die zu der vorgegebenen Tiefe gehört, wenn das Doppler-Signal nicht mehr detektiert wird, und
Anpassen der Positionen der nachfolgenden Abtastvolumina in Inkrementen entlang einer zweiten Richtung, bis ein Doppler-Signal nicht mehr detektiert wird, wobei die zweite Richtung der ersten Richtung entgegengesetzt ist.

11. Verfahren nach Anspruch 8, das weiterhin Folgendes umfasst:
Berechnen einer mittleren Doppler-Geschwindigkeit von jedem der mindestens einen Teile der Positionen der nachfolgenden Abtastvolumina;
Synchronisieren der mittleren Geschwindigkeit unter Verwendung einer arteriellen Pulsatilität in dem Doppler-Spektrum, wenn die Pulsatilität detektiert wird; und
Anwenden von Doppler-Winkelkorrektur unter Verwendung einer Orientierung des Gefäßes.

12. Ultraschall-Bildgebungssystem (10), das Folgendes umfasst:
ein Matrixwandlerarray (120) zum Aussenden von Ultraschallwellen in eine Region eines Körpers mit einem Gefäß und Empfangen von Echos in Reaktion darauf, wobei die Echos der Blutströmung durch das Gefäß zugehörig sind; und
einen Prozessor (100), der betriebsfähig mit dem Matrixwandlerarray gekoppelt ist, wobei der Prozessor im Anschluss an das Empfangen von Echos in einem anfänglichen Abtastvolumen zu den Echos gehörige Positionen der Abtastvolumina (250) anpasst, wobei das anfängliche Abtastvolumen basierend auf einer typischen Lage des anfänglichen Gefäßes im Körper auf eine Anfangsposition und -tiefe eingestellt ist, wobei das Anpassen der nachfolgenden Positionen der Abtastvolumina auf einem Verfolgungsalgorithmus zum Verfolgen eines Pfads des Gefäßes durch Bewegen der Positionen der nachfolgenden Abtastvolumina in Tiefe-Inkrementen entlang des Gefäßes basiert; wobei der Prozessor die Ultraschallwellen elektronisch auf die nachfolgenden Positionen der Abtastvolumina lenkt; und wobei der Prozessor eine Gefäßwand bei jeder der nachfolgenden Positionen der Abtastvolumina basierend auf einem Doppler-Spektrum ermittelt, das bei jeder der nachfolgenden Positionen der Abtastvolumina erfasst wurde.

13. System nach Anspruch 12, wobei der Prozessor eine Mittellinie des Gefäßes basierend auf einer Stärke eines Doppler-Signals an jeder der nachfolgenden Positionen der Abtastvolumina ermittelt.

14. System nach Anspruch 12, weiterhin mit einer Halterungskonstruktion (150) zum Positionieren des Matrixwandlerarrays (120) in Bezug auf die Region des Körpers mit dem Gefäß.

15. System nach Anspruch 12, weiterhin mit einer Anzeigevorrichtung (170), die mit dem Prozessor (100) in Kommunikation steht, wobei die Anzeigevorrichtung eine Gefäßkarte darstellt, welche durch den Prozessor basierend mindestens teilweise auf der Ermittlung der Gefäßwand bei jeder der anfänglichen und nachfolgenden Positionen der Abtastvolumina (250) erzeugt wurde.

## Revendications

1. Procédé d'imagerie de vaisseaux, le procédé comprenant :
le positionnement d'une sonde à ultrasons (120) près d'une fenêtre temporale d'un corps afin de saisir une image Doppler d'un vaisseau initial à examiner ;
la transmission d'ondes à ultrasons dans une zone correspondante du corps dans laquelle se trouve le vaisseau et recevant des échos en réponse, les échos étant associés à un flux sanguin à travers le vaisseau ;
l'ajustage de positions de volumes d'échantillon (250) associés aux échos consécutifs à la réception d'échos dans un volume d'échantillon initial, le volume d'échantillon initial étant établi dans une position initiale et une profondeur, sur la base d'un emplacement typique du vaisseau initial dans le corps, l'ajustement des positions successives des volumes d'échantillon étant basé sur un algorithme de traçage permettant de tracer un chemin du vaisseau, en déplaçant les positions des volumes d'échantillon successifs par incréments de profondeur le long du vaisseau ;
l'orientation électronique des ondes à ultrasons dans les positions successives des volumes d'échantillon ; et
la détermination d'une paroi du vaisseau à chacune des positions successives des volumes d'échantillon, sur la base d'un spectre Doppler capturé à chacune des positions successives des volumes d'échantillon.

2. Procédé selon la revendication 1, comprenant en outre :
l'ajustage des positions successives des volumes d'échantillons par incréments le long d'une première direction jusqu'à ce qu'une force d'un signal Doppler soit inférieure à un certain seuil ; et
le retour à une première position des positions successives des volumes d'échantillon quand la force du signal Doppler a chuté en-deçà du seuil.

3. Procédé selon la revendication 2, comprenant en outre l'ajustage des positions successives des volumes d'échantillon par incréments le long d'une seconde direction jusqu'à ce qu'une force du signal Doppler soit inférieure au seuil, la seconde direction étant opposée à la première direction.

4. Procédé selon la revendication 1, comprenant en outre :
la construction d'une carte de vaisseau, en reliant au moins une partie des positions initiales et successives des volumes d'échantillon afin de construire un squelette de vaisseau ;
l'intégration des données électriques du Doppler de chacune desdites au moins une partie des positions initiales et successives des volumes d'échantillon le long du squelette de vaisseau ; et
l'ajustage d'une luminosité du squelette de vaisseau pour représenter les données électriques de Doppler.

5. Procédé selon la revendication 4, comprenant en outre au moins un élément parmi l'interpolation et le lissage entre les points le long du squelette de vaisseau.

6. Procédé selon la revendication 4, comprenant en outre :
le calcul d'une vitesse moyenne de Doppler de chacune d'au moins une partie des positions initiale et successive des volumes d'échantillon ; et
la synchronisation de la vitesse moyenne en utilisant une pulsatilité artérielle dans le spectre de Doppler quand la pulsatilité est détectée.

7. Procédé selon la revendication 6, comprenant en outre l'application d'une correction d'angle Doppler en utilisant une orientation du vaisseau.

8. Procédé de réalisation d'une imagerie trans-crânienne, le procédé comprenant :
la saisie d'une image Doppler d'un vaisseau initial dans la région trans-crânienne ;
le positionnement d'un volume d'échantillon Doppler initial (250) à proximité du vaisseau initial à une profondeur prédéterminée, en utilisant l'image Doppler comme guide ;
l'ajustage des positions des volumes d'échantillon successifs sur la base d'un algorithme de traçage, afin de tracer un chemin du vaisseau en déplaçant les positions des volumes d'échantillon successifs par incréments de profondeur le long du vaisseau ;
l'orientation électronique des ondes à ultrasons aux positions des volumes d'échantillon successifs ; et
la détermination d'une ligne centrale et d'une paroi du vaisseau pour au moins une partie des positions des volumes d'échantillon successifs, sur la base d'un spectre Doppler associé à un flux sanguin à travers le vaisseau, qui est saisi au niveau de chacune des positions des volumes d'échantillon successifs, la détermination de la ligne centrale et de la paroi étant déterminée sur la base de la force du signal Doppler.

9. Procédé selon la revendication 8, comprenant en outre :
la construction d'une carte de vaisseau en reliant au moins une partie des positions des volumes d'échantillon successifs afin de construire un squelette de vaisseau ;
l'intégration des données électriques de Doppler de chacune d'au moins une partie des positions des volumes d'échantillon successifs, le long du squelette de vaisseau ; et
l'ajustage de la luminosité du squelette de vaisseau afin de représenter les données électriques de Doppler.

10. Procédé selon la revendication 8, comprenant en outre :
l'ajustage des positions des volumes d'échantillon successifs par incréments le long d'une première direction jusqu'à ce qu'un signal Doppler ne soit plus détecté ;
le retour à une première position des volumes d'échantillon associés à une profondeur prédéterminée quand le signal de Doppler n'est plus détecté ; et
l'ajustage des positions des volumes d'échantillon successifs par incréments le long d'une seconde direction jusqu'à ce qu'un signal Doppler ne soit plus détecté, la seconde direction étant opposée à la première direction.

11. Procédé selon la revendication 8, comprenant en outre :
le calcul d'une vitesse moyenne de Doppler de chacune desdites au moins une partie des positions des volumes d'échantillon successifs ;
la synchronisation de la vitesse moyenne en utilisant une pulsatilité artérielle dans le spectre Doppler quand la pulsatilité est détectée ; et
l'application d'une correction d'angle Doppler en utilisant une orientation du vaisseau.

12. Système d'imagerie à ultrasons (10) comprenant :
un réseau de transducteur de matrice (120) pour transmettre les ondes à ultrasons dans une zone du corps dotée d'un vaisseau et recevant des échos en réponse, les échos étant associés au flux sanguin à travers le vaisseau ; et
un processeur (100) raccordé opérationnellement au réseau de transducteur de matrice, dans lequel le processeur ajuste des positions des volumes d'échantillon (250) associés aux échos successifs à la réception d'échos dans un volume d'échantillon initial, le volume d'échantillon initial étant établi à une position et à une profondeur initiales, sur la base d'un emplacement typique du vaisseau initial dans le corps, l'ajustage des positions successives des volumes d'échantillon étant basé sur un algorithme de traçage permettant de tracer un chemin du vaisseau, en déplaçant les positions des volumes d'échantillon successifs par incréments de profondeur, le long du vaisseau, dans lequel le processeur dirige électroniquement les ondes à ultrasons à des positions successives des volumes d'échantillon, et dans lequel le processeur détermine une paroi du vaisseau à chacune des positions successives des volumes d'échantillon, sur la base d'un spectre Doppler capturé à chacune des positions successives des volumes d'échantillon.

13. Système selon la revendication 12, dans lequel le processeur détermine une ligne centrale du vaisseau, sur la base d'une force d'un signal Doppler à chacune des positions successives des volumes d'échantillon.

14. Système selon la revendication 12, comprenant en outre une structure de support (150) pour positionner le réseau de transducteur de matrice (120) relativement à la zone du corps dans laquelle se trouve le vaisseau.

15. Système selon la revendication 12, comprenant en outre un dispositif d'affichage (170) en communication avec le processeur (100), dans lequel le dispositif d'affichage présente une carte de vaisseau générée par le processeur, sur la base au moins en partie de la détermination de la paroi du vaisseau à chacune des positions initiale et successives du volume d'échantillon (250).
